# FASCICULE DE BREVET EUROPEEN

(11) **EP 3 897 551 B1**
(45) Date de publication et mention de la délivrance du brevet: **31.01.2024**
(21) Numéro de dépôt: 19845586.7
(22) Date de dépôt: 13.12.2019
(51) Int. Cl.: A61K 8/34, A61K 8/73, A61Q 19/00, A61K 8/02, A61K 8/60

(54) **COMPOSITION FILMOGÈNE À BASE D'AMIDON DE LÉGUMINEUSE À USAGE COSMÉTIQUE**
FILMBILDENDE ZUSAMMENSETZUNG AUF DER BASIS VON LEGUMINOSESTÄRKE ZUR KOSMETISCHEN VERWENDUNG
FILM-FORMING COMPOSITION BASED ON LEGUMINOUS STARCH FOR COSMETIC USE

(30) Priorité: 17.12.2018 FR 1873080
(43) Date de publication de la demande: 27.10.2021
(73) Titulaire: ROQUETTE FRERES, 62136 Lestrem (FR)
(72) Inventeur: MENTINK, Léon, 59000 LILLE (FR); LE BIHAN, Grégory, 62232 ANNEZIN (FR); BIRRAUX, Jérémie, 75013 PARIS (FR)
(74) Mandataire: Plasseraud IP
(86) Numéro de dépôt international: PCT/FR2019/053059
(87) Numéro de publication internationale: WO 2020/128253

(56) Documents cités:
- WO-A1-2014/155015
- WO-A1-2016/168574
- WO-A2-2013/167835
- FR-A1- 2 862 654
- KR-A- 20130 045 724

## Description

### Domaine technique

L'invention a pour objet une composition filmogène, une composition cosmétique de soins ou de maquillage la contenant, un procédé cosmétique de maquillage et de soins ainsi que différentes utilisations cosmétiques.

### Technique antérieure

Les agents filmogènes sont connus en cosmétique pour leur capacité à produire un film continu sur la peau, les cheveux ou les ongles.

Typiquement, les compositions cosmétiques capillaires contiennent des polymères filmogènes permettant d'obtenir un film gainant sur les cheveux. Ces propriétés sont utilisées notamment pour renforcer la tenue de la coiffure.

Appliqués sur la peau, les agents filmogènes s'opposent à l'évaporation de l'eau et réduisent ainsi les pertes en eau. Ces agents permettent d'apporter à la peau une hydratation profonde et durable. Ils peuvent permettre aussi de protéger la peau en ayant par exemple un effet barrière aux pollutions, notamment à la pollution des microparticules atmosphériques, et aux rayons ultra-violets. Dans le maquillage et typiquement dans les mascaras, les agents filmogènes augmentent significativement le recourbement et l'allongement des cils. Dans la protection solaire, les agents filmogènes permettent de former des films bloquant les rayonnements UV, ou réduisant la quantité de rayonnement qui atteint la peau.

Des polymères d'origine naturelle ont également été utilisés en cosmétique pour leurs propriétés biomécaniques et filmogènes puissantes conférant une efficacité de seconde peau protectrice. Les polymères d'origine naturelle sont ceux extraits ou obtenus à partir de ressources naturelles renouvelables, c'est-à-dire soit végétales, comme les amidons et leurs dérivés, les celluloses et leurs dérivés, les gommes issues de graines ou d'exsudats de plantes, les gommes extraites d'algues, soit issues de la fermentation de micro-organismes tels que les champignons, les bactéries, comme les gommes de xanthane, les carraghénanes,...

Il a été décrit dans le document WO 2013/167835 des compositions cosmétiques comprenant des mélanges d'ingrédients qui procurent de l'élasticité au produit cosmétique de type masque appliqué à la surface de la peau naturellement sollicitée par des déformations. Typiquement, des compositions cosmétiques contenant un mélange apte à former un film élastique constitué d'au moins un hydrolysat d'amidon, d'au moins un polyol, d'au moins un polymère choisi parmi les alcools polyvinyliques, les polymères et copolymères de vinylpyrrolidone et les latex, et éventuellement d'eau sont décrites.

Ces agents ou compositions filmogènes contiennent encore une part importante de polymères synthétiques, et s'avèrent difficiles à rincer ou à retirer. Par ailleurs, il est difficile d'obtenir des compositions présentant une bonne tenue mécanique lors de l'application.

Il est ainsi nécessaire de mettre au point une composition cosmétique filmogène d'origine naturelle présentant un bon compromis entre une bonne tenue mécanique lors de l'application et une bonne aptitude à pouvoir être éliminée postérieurement à l'application, par exemple par lavage.

### Résumé de l'invention

Il est du mérite de la Demanderesse d'avoir mis au point des nouvelles compositions filmogènes à usage cosmétique d'origine naturelle présentant une bonne tenue mécanique lors de l'application ainsi qu'une bonne aptitude à une élimination possible par simple rinçage ou lavage.

Ces compositions filmogènes permettent de conférer également aux matières kératiniques d'excellentes propriétés quant à l'unification du teint, l'uniformité du teint, l'homogénéité du teint, l'obtention d'un teint matifié, le lissage de la peau, la protection des zones sensibilisées d'une fibre capillaire, le lissage des écailles des fibres capillaires, le gainage des fibres capillaires, le recourbement et l'allongement des cils, la résistance à des chocs mécaniques. Un autre effet est l'effet barrière aux agressions extérieures telles que les micropolluants atmosphériques, les UV, les oxydants, en limitant ou empêchant leur migration vers le derme ou les matières kératiniques.

La présente invention concerne également des compositions cosmétiques de protection, de soin ou de maquillage comprenant une composition filmogène d'origine naturelle ainsi qu'un procédé de maquillage ou de soin des matières kératiniques comprenant l'application sur lesdites matières kératiniques d'au moins une composition cosmétique selon la présente invention.

### Exposé de l'invention

Un premier objet de l'invention concerne une composition filmogène comprenant de:
- 1 % à 20 % d'au moins un amidon de légumineuse hydrolysé et alkylé,
- 1 % à 20% d'au moins un polyol, et
- 1 % à 10 % d'au moins un carraghénane,
les pourcentages étant exprimés en poids par rapport au poids total de la composition filmogène.

D'une manière générale, on entend par « composition filmogène », une composition comprenant au moins un polymère (agent filmogène), ledit polymère étant apte à former un film essentiellement continu en présence d'un solvant, en particulier d'eau. Au sens de la présente invention, les compositions filmogènes sont en particulier des compositions filmogènes d'origine naturelle à base d'amidon, en ce sens que l'amidon est utilisé en tant qu'agent filmogène principal.

Par « légumineuse » au sens de la présente invention, on entend toute plante appartenant aux familles des césalpiniacées, des mimosacées ou des papilionacées et notamment toute plante appartenant à la famille des papilionacées comme par exemple, le pois, le haricot, la fève, la fèverole, la lentille, ou le lupin.

Selon un mode de réalisation préféré, l'amidon de légumineuse est un amidon de pois.

L'amidon de légumineuse selon l'invention est obtenu à partir d'un amidon présentant une teneur en amylose comprise de 25 % à 45 %, de préférence de 30 % à 45%, et de préférence encore de 35 % à 40 %, les pourcentages étant exprimés en poids sec par rapport au poids sec d'amidon de légumineuse, et déterminés avant tout traitement ultérieur tel qu'une hydrolyse et/ou une alkylation dudit amidon.

L'amidon de légumineuse selon l'invention est un amidon modifié. Il s'agit d'un amidon hydrolysé et alkylé, de préférence hydroxypropylé.

Ainsi, selon un mode de réalisation préféré, l'amidon selon l'invention est un amidon de légumineuse hydrolysé et hydroxypropylé.

On entend par « amidon hydroxypropylé » au sens de la présente invention, un amidon substitué par des groupements hydroxypropyle par toute technique connue de l'homme du métier, par exemple par réaction d'éthérification avec l'oxyde de propylène, présentant en particulier une teneur en groupements hydroxypropyle comprise entre 0,1 et 20% en poids sec par rapport au poids sec d'amidon hydroxypropylé, préférentiellement entre 1 et 10%, plus préférentiellement entre 5 et 9%, et en particulier proche de 7%. Cette teneur est en particulier déterminée par spectrométrie par Résonance Magnétique Nucléaire du proton, en particulier selon la norme EN ISO 11543:2002 F.

On entend par « amidon hydrolysé » au sens de la présente invention, un amidon ayant subi une opération d'hydrolyse, c'est-à-dire une opération visant à réduire sa masse moléculaire moyenne. L'homme du métier sait comment obtenir de tels amidons, par exemple par des traitements chimiques tels que l'oxydation et les traitements acides, ou encore par des traitements enzymatiques. L'homme du métier ajustera naturellement le niveau de fluidification de l'amidon, en fonction de la viscosité souhaitée pour la composition filmogène.

L'amidon de légumineuse hydrolysé et alkylé selon l'invention peut également comprendre une ou plusieurs autres modifications physiques et/ou chimiques, du moment que lesdites modifications n'interfèrent pas avec les propriétés désirées dudit amidon au sein de la composition filmogène selon l'invention. Un exemple de modification chimique est notamment la réticulation.

Selon un mode de réalisation particulier, l'amidon hydrolysé et alkylé de la composition filmogène selon l'invention peut en outre présenter d'autres modifications, et peut par exemple avoir subi des traitements physiques, notamment choisis parmi les opérations connues de gélatinisation, pré-gélatinisation, d'extrusion, d'atomisation ou de séchage, les opérations de traitement par micro-ondes ou ultrasons, de plastification ou de granulation.

En particulier, l'amidon après alkylation et hydrolyse sera de préférence non granulaire. Il sera avantageusement rendu soluble dans l'eau par toute technique connue afin que la composition filmogène présente de très bonnes propriétés filmogènes.

Ainsi, selon un mode de réalisation avantageux, l'amidon selon l'invention est caractérisé en ce qu'il est rendu soluble. Il peut être rendu soluble par toute technique connue de l'homme du métier, notamment par traitement thermique et/ou mécanique, par exemple par une opération de cuisson en milieu aqueux, éventuellement suivie d'une étape de séchage lorsque l'obtention d'un produit pulvérulent est souhaitée. L'opération visant à rendre soluble l'amidon peut tout à fait intervenir avant l'introduction dudit amidon dans la composition filmogène, de manière indifférente avant ou après l'alkylation et/ou l'hydrolyse de l'amidon, ou encore après son introduction dans la composition filmogène, par exemple en cuisant la composition filmogène au moment de sa mise en oeuvre.

L'amidon hydrolysé et alkylé selon l'invention a de préférence un poids moléculaire moyen en poids compris de 1 à 2 000 kDa, de préférence de 10 à 1 000 kDa, tout préférentiellement de 20 à 1 000 kDa, et encore plus préférentiellement de 100 à 1 000 kDa. Par exemple, le poids moléculaire peut être compris de 200 à 800 kDa, de 200 à 500 kDa, de 200 à 400 kDa ou encore de 200 à 300 kDa. Le poids moléculaire moyen en poids étant déterminé par HPSEC-MALLS (chromatographie d'exclusion de taille à haute performance couplée en ligne avec une détection par diffusion de lumière laser à angles multiples).

Des amidons hydrolysés et hydroxypropylés adaptés pour la composition filmogène selon l'invention sont commercialement disponibles et sont proposés par la Demanderesse sous la marque Beauté by Roquette^{®} ST720.

La composition filmogène comprend de 1 % à 20 %, de préférence de 1 % à 15 %, et tout particulièrement de 1 % à 10 % en poids d'amidon de légumineuse hydrolysé et alkylé. Les pourcentages en poids étant exprimés par rapport au poids total de la composition filmogène.

La composition filmogène selon l'invention comprend également un ou plusieurs polyols. Le polyol est utilisé dans la composition filmogène en tant qu'agent plastifiant.

N'importe quel polyol connu de l'homme du métier peut être utilisé dans la composition filmogène selon l'invention.

Selon un mode de réalisation particulier, le polyol est choisi parmi le groupe constitué du maltitol, mannitol, xylitol, érythritol, sorbitol, isosorbide, glycérol ou glycérine, glucose, saccharose, polydextrose, les sirops de glucose hydrogénés, les dextrines, les les maltodextrines, les sirops de glucose, et leurs mélanges. De préférence, le polyol est choisi parmi le glycérol et le sorbitol.

La composition filmogène comprend de 1 % à 20 %, de préférence de 1 % à 15 %, et tout particulièrement de 1 % à 10 % en poids de polyols, les pourcentages en poids étant exprimés par rapport au poids total de la composition filmogène.

La composition filmogène selon l'invention comprend également au moins un carraghénane. Le carraghénane est utilisé en tant qu'agent épaississant au sein de la composition filmogène.

Selon un mode de réalisation préféré, le carraghénane est choisi parmi le iota carraghénane, le lambda carraghénane, le kappa carraghénane, et leurs mélanges.

Le carraghénane est présent dans la composition filmogène selon une quantité comprise de 1 à 10 %, de préférence de 1 % à 7 %, et tout préférentiellement de 1 % à 5 % en poids. Les pourcentages en poids étant exprimés par rapport au poids total de la composition filmogène.

Selon un mode de réalisation particulier, la composition filmogène peut en outre comprendre un agent tampon choisi parmi les sels de calcium, ou de potassium, comme le chlorure de calcium, le chlorure de potassium, le phosphate de potassium monobasique et le phosphate de calcium dibasique.

Selon un mode de réalisation particulier, la composition filmogène selon l'invention est une composition aqueuse. Selon ce mode de réalisation particulier, elle présente une teneur en matière sèche comprise de 1 à 50 % en poids, préférentiellement de 2 à 40 % en poids, et plus préférentiellement de 5 à 25 % en poids.

Selon un mode de réalisation particulier, la composition filmogène peut en outre comprendre :
- d'autres agents filmogènes que l'amidon hydrolysé et alkylé utilisé conformément à l'invention, par exemple des polymères d'origine naturelle tels que l'hydroxypropyl méthyl-cellulose (HPMC), l'hydroxypropyl-cellulose (HPC), ou des polymères synthétiques tels que l'alcool polyvinylique (PVA), - et/ou
- d'autres plastifiants que les polyols selon l'invention, comme du polyéthylène glycol, du triéthylcitrate, du polysorbate, ou encore des cires comme la cire de Carnauba, ou de l'huile de ricin hydrogénée.

Afin de faciliter la mise en oeuvre, il est cependant préférable de minimiser la quantité des matières première à employer. Par conséquent, et de manière préférentielle, l'amidon hydrolysé et alkylé ainsi que le polyol représentent dans la composition filmogène selon l'invention l'essentiel, et préférentiellement la totalité, des agents filmogènes et des plastifiants respectivement.

Avantageusement, les quantités des différents composés de la composition filmogène selon l'invention peuvent également être exprimées en fonction de la matière sèche totale de ladite composition filmogène. En effet, c'est le rapport entre l'agent filmogène et l'agent plastifiant qui détermine notamment la plasticité et donc la qualité du film formé.

Ainsi, la composition filmogène selon l'invention comprend :
- de 20 à 60 %, préférentiellement de 30% à 50 % d'au moins un amidon de légumineuse hydrolysé et alkylé,
- de 20 à 60 %, préférentiellement de 30 % à 50 %, d'au moins un polyol, et
- de 5 à 25 %, préférentiellement de 10% à 20 %, d'au moins un carraghénane. Les pourcentages étant exprimés en poids sec par rapport au poids sec total de la composition filmogène.

Selon un mode de réalisation avantageux, l'amidon hydrolysé et alkylé, le polyol et le carraghénane représentent ensemble entre 20 % et 100 % en poids sec par rapport au poids sec total de la composition filmogène, de préférence entre 30 % et 99 %, de préférence encore entre 50 et 99 %, et tout préférentiellement de 95 à 99 % en poids sec par rapport au poids sec total de la composition filmogène.

Selon un mode de réalisation particulier, la composition filmogène contient :
- de 20 à 60 %, préférentiellement de 30% à 50 % d'un amidon de légumineuse hydrolysé et alkylé,
- de 20 à 60 %, préférentiellement de 30 % à 50 % d'au moins un polyol, et
- de 5 à 25 %, préférentiellement de 10% à 20 % d'un carraghénane,
- optionnellement de 0,01 à 0,5 % d'un agent tampon choisi parmi les sels de calcium ou de potassium, préférentiellement choisi parmi le chlorure de calcium, le chlorure de potassium, et le phosphate de potassium monobasique,
- optionnellement de 0 à 15 % d'un autre ingrédient, de préférence de 0 à 5 %, de préférence encore de 0 à 3 %, et tout particulièrement de 0 à 1 %.

Les pourcentages étant exprimés en poids sec par rapport au poids sec total de la composition, leur somme totale étant égale à 100 %.

Les compositions filmogènes de l'invention peuvent en effet contenir d'autres ingrédients pour autant qu'ils n'interfèrent pas avec les propriétés souhaitées desdites compositions filmogènes. Ces autres ingrédients peuvent par exemple être des substances tensioactives, des charges opaques, des conservateurs, des agents antimicrobiens, des édulcorants, des substances aromatisantes, des colorants, des azurants, ou encore de lubrifiants.

La présente invention concerne également l'utilisation cosmétique de la composition filmogène selon la présente invention telle que précédemment décrite.

Aussi, la présente invention concerne l'utilisation non thérapeutique d'une composition filmogène selon la présente invention pour conférer aux matières kératiniques au moins une propriété choisie parmi l'unification du teint, l'uniformité du teint, l'homogénéité du teint, l'obtention d'un teint matifié, l'effet barrière, le lissage de la peau, la protection des zones sensibilisées d'une fibre capillaire, le lissage des écailles des fibres capillaires, le gainage des fibres capillaires, le recourbement et l'allongement des cils, la résistance à des chocs mécaniques.

En effet, par ses propriétés, la composition filmogène au sens de la présente invention va permettre d'unifier, d'uniformiser, d'homogénéiser, de matifier le teint. Les propriétés filmogènes de la composition selon la présente invention telle que précédemment décrite vont permettre, lorsque la composition selon la présente invention sera appliquée sur les matières kératiniques, préférentiellement sur la peau du visage ou du corps de lisser la peau, de masquer les imperfections afin d'obtenir un teint plus unifié, uniforme, homogène et matifié.

La composition filmogène au sens de la présente invention va protéger les zones sensibilisées d'une fibre capillaire, lisser les écailles des fibres capillaires, gainer les fibres capillaires. Ces propriétés sont obtenues par la formation d'un film à la surface de la fibre capillaire.

La composition filmogène au sens de la présente invention permettre le recourbement et l'allongement des fibres kératiniques, préférentiellement des cils.

La composition filmogène au sens de la présente invention va conférer un effet barrière aux matières kératiniques. En effet, la composition filmogène selon la présente invention va protéger les matières kératiniques des agressions extérieures telles que les micropolluants atmosphériques, les UV, les oxydant, en limitant ou empêchant leur migration vers le derme ou le coeur de la fibre capillaire. Ainsi, on entend par « effet barrière » au sens de la présente invention la protection des matières kératiniques vis-à-vis des agressions extérieures.

La composition filmogène est telle que précédemment définie. La présente invention concerne également une composition cosmétique de maquillage, de soin ou de protection des matières kératiniques comprenant dans un milieu physiologiquement acceptable, une composition filmogène comprenant au moins un amidon de légumineuse hydrolysé et alkylé, au moins un polyol, au moins un carraghénane.

La composition filmogène et notamment les amidons de légumineuse hydrolysés et alkylés, les polyols, les carraghénanes sont tels que précédemment définis.

La composition cosmétique peut en outre comprendre un agent tampon tel que précédemment défini.

Selon un mode de réalisation, la composition filmogène présente dans la composition cosmétique de maquillage, de soin ou de protection des matières kératiniques, comprend:
- 1 % à 20 % d'au moins un amidon de légumineuse hydrolysé et alkylé, préférentiellement d'au moins un amidon de pois hydrolysé et hydroxypropylé,
- 1 % à 20% d'au moins un polyol, préférentiellement choisi parmi le sorbitol et le glycérol, et
- 1 % à 10 % d'au moins un carraghénane,
les pourcentages étant exprimés en poids par rapport au poids total de la composition filmogène.

Le milieu physiologiquement acceptable est de préférence cosmétiquement ou dermatologiquement acceptable. On entend par « milieu physiologiquement acceptable » ou « cosmétiquement acceptable » ou « dermatologiquement acceptable » au sens de la présente invention, tout milieu qui ne présente pas d'effets secondaires délétères et en particulier qui ne produit pas de rougeurs, d'inflammation, d'échauffement, de tiraillements ou de picotements inacceptables pour un utilisateur de produits cosmétiques. Le milieu est ainsi compatible avec les matières kératiniques d'êtres humains.

Typiquement, les matières kératiniques seront la peau, les muqueuses, le cuir chevelu ou les cheveux, le système pileux, les cils, les sourcils, les ongles.

Selon un mode de réalisation, la composition filmogène au sens de la présente invention va conférer à la composition cosmétique de maquillage ou de soin au moins une propriété choisie parmi l'unification, l'uniformité, l'homogénéité du teint, l'obtention d'un teint matifié. Les propriétés filmogènes de la composition vont permettre, lorsque la composition sera appliquée sur les matières kératiniques, préférentiellement sur le visage ou le corps de lisser la peau, de masquer les imperfections afin d'obtenir un teint plus unifié, uniforme, homogène et matifié.

Selon un autre mode de réalisation, la composition filmogène au sens de la présente invention confère à la composition cosmétique de maquillage ou de soin des propriétés de protection des zones sensibilisées d'une fibre capillaire, de lissage des écailles des fibres capillaires, de gainage des fibres capillaires. Ces propriétés sont obtenues par la formation d'un film à la surface de la fibre capillaire.

Selon un autre mode de réalisation, la composition filmogène au sens de la présente invention va conférer à la composition cosmétique de maquillage ou de soin des propriétés de recourbement et d'allongement des fibres kératiniques, préférentiellement des cils.

Selon un autre mode de réalisation, la composition filmogène au sens de la présente invention confère un effet barrière aux matières kératiniques. On entend par effet barrière la protection offerte par la présente composition filmogène aux matières kératiniques, en empêchant ou limitant la migration vers le derme ou le coeur de la fibre capillaire d'agents responsables d'aux agressions extérieures telles que les micropolluants atmosphériques, les matières ou composés oxydants atmosphériques, ou les rayonnements du soleil, comme les ultra-violets.

Selon un autre mode de réalisation, la composition filmogène au sens de la présente invention confère à la composition cosmétique de maquillage ou de soin des propriétés de résistances à des chocs mécaniques, par la formation d'un film à la surface de la matière kératinique, typiquement à la surface d'un ongle.

Ainsi, la présente invention concerne également l'utilisation non thérapeutique d'une composition cosmétique de maquillage, de protection ou de soin des matières kératiniques telle que précédemment définie destinée à conférer aux matières kératiniques au moins une propriété choisie parmi l'unification du teint, l'uniformité du teint, l'homogénéité du teint, l'obtention d'un teint matifié, un effet barrière, le lissage de la peau, la protection des zones sensibilisées d'une fibre capillaire, le lissage des écailles des fibres capillaires, le gainage des fibres capillaires, le recourbement et l'allongement des cils, la résistance à des chocs mécaniques

La composition cosmétique de maquillage, de protection ou de soin selon l'invention peut se présenter sous toutes les formes galéniques utilisées dans le domaine cosmétique, normalement utilisées pour une application topique telles que des émulsions (en particulier crème ou lait), des gels, des crèmes, des solutions, des dispersions du type lotion ou sérum, des émulsions de consistance liquide ou semi-liquide obtenues par dispersion d'une phase grasse dans une phase aqueuse (émulsion huile-dans-eau, H/E) ou inversement (émulsion eau-dans-huile, E/H), des suspensions, des lotions, des microémulsions, des microcapsules, des microparticules ou des dispersions vésiculaires de type ionique et/ou non ionique, des vernis, des huiles corporelles, des masques, des pommades, des onguents, des solutions concentrées. Ces formes galéniques sont obtenues selon les méthodes usuelles connues de l'homme du métier. Ces formes galéniques contiennent en outre des excipients et supports usuels et acceptables sur le plan cosmétologique.

A titre illustratif, le milieu physiologiquement acceptable pourra comprendre une phase grasse et/ou une phase aqueuse.

### Phase grasse

La phase grasse peut renfermer au moins une huile.

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

La phase grasse liquide comprend avantageusement une ou plusieurs huiles non volatiles qui procurent un effet émollient sur la peau. On peut citer les esters gras tels que l'isononoate de cetearyl, l'isononoate d'isotridecyl, l'isostearate d'isostearyl, l'isostearate d'isopropyl, le myristate d'isopropyl, le palmitate d'isopropyl, stéarate de butyle, le laurate d'hexyle, l'isononate d'isononyle, le palmitate de 2-éthyl-hexyle, le laurate de 2-hexyldécyle, le palmitate de 2-octyl décyle, le myristate ou la lactate de 2-octyldodécyle, le succinate de 2-diéthyl hexyle, le malate de diisostéaryle, la tracétine , la tricprine , les triglycérides d'acide caprylique/caprique, le mélange de caprate et caprylate de coco, les benzoates d'alcools en C12 à C15, les esters de glycol tels que le butylène glycol cocoate, le triisostéarate de glycérine, l'acetate de tocopherol, les acides gras supérieurs tels que l'acide myristique, l'acide palmitique, l'acide stéarique, l'acide béhénique, l'acide oléique, l'acide linoléique, l'acide linolénique ou l'acide isostéarique, les alcools gras supérieurs tel que l'alcool oléique, les huiles végétales comme l'huile d'avocat, l'huile de camélias, l'huile de noisette, l'huile de tsubaki, l'huile de noix de cajou, l'huile d'argan, l'huile de soja, l'huile de pépins de raisin, l'huile de sésame, l'huile de mais, l'huile de germes de blé, l'huile de colza, l'huile de tournesol, l'huile de coton, l'huile de jojoba, l'huile d'arachide, l'huile d'olive et leurs mélanges, les beurres végétaux comme le beurre de karité, le beurre de camellia.

Ces huiles peuvent également être des huiles de type hydrocarbures ou siliconnées telles que l'huile de paraffine, de squalane, la vaseline, les diméthyls siloxanes et leurs mélanges.

La phase grasse liquide peut également comprendre des huiles volatiles. Par huile volatile, on entend une huile susceptible de s'évaporer de la peau, en moins d'une heure à température ambiante et pression atmosphérique. Les huiles volatiles peuvent être par exemple choisies parmi les huiles de silicones ou triglycérides d'acides gras courts pour réduire le toucher gras.

La phase grasse de la composition selon l'invention peut en outre avantageusement comprendre au moins un agent structurant de phase grasse tel qu'un gélifiant lipophile, une cire et/ou un composé pâteux.

Par « cire », on entend un corps gras à changement d'état liquide / solide réversible, ayant une température de fusion supérieure à 30°C et généralement inférieure à 90°C, qui est liquide dans les conditions de préparation de la composition et présente à l'état solide une organisation cristalline anisotrope. Les cires utilisées selon l'invention peuvent être constituées de cires polaires ou apolaires ou d'un mélange des deux. Par « apolaire », on entend une cire ne renfermant que des atomes de carbone, d'hydrogène et/ou de phosphore et en particulier un hydrocarbure.

Les cires polaires peuvent notamment être choisies parmi les cires animales, les cires végétales et les cires synthétiques ou de silicone renfermant des groupements polaires tels que les esters. On peut ainsi mentionner les cires de Carnauba, de Candelilla, d'abeille (Cera alba), d'insecte de Chine (Ericerus pela); du Japon, de Sumac, de Montan, les triesters d'acides en C8-C20 et de glycérine tels que le tribéhénate de glycérine, le stéarate de glycol acétylé commercialisé notamment par la société VEVY sous la dénomination commerciale CETACENE, et leurs mélanges. Ces cires peuvent notamment être utilisées sous forme prédispersée dans une huile, comme c'est le cas du mélange de cire de candelilla et d'huile de graine de jojoba.

On entend par "composé pâteux" des corps gras lipophiles qui, comme les cires, sont capables de subir un changement d'état liquide/solide réversible et ont à l'état solide une organisation cristalline anisotrope, mais qui se différencient des cires par le fait qu'ils renferment, à une température de 23°C, une fraction liquide et une fraction solide.

### Phase aqueuse

La composition cosmétique selon l'invention peut, en outre, comprendre une phase aqueuse comprenant de l'eau et optionnellement, un ou plusieurs solvants organiques miscible à l'eau, et/ou un ou plusieurs agents de rhéologie.

L'agent de rhéologie peut notamment être un agent épaississant de la phase aqueuse, un agent gélifiant ou un agent suspensif, tels que par exemple les gommes issues de plantes comme la gomme arabique, la gomme de konjac , la gomme de guar ou leurs dérivés ; les gommes extraits d'algues comme les alginates; les gommes issues d'une fermentation microbienne comme les xanthanes, les mannanes , les scléroglucanes ou leurs dérivés ; la cellulose et ses dérivés comme la carboxyméthylcellulose ou l'hydroxyéthylcellulose ; l'amidon et ses dérivés comme en particulier les amidons modifiées, notamment acétylés, carboxyméthylés, octénylsuccinates ou hydroxypropylés ; les polymères synthétiques comme les polyacide-acryliques ou les carbomères.

### Tensioactifs

La composition cosmétique selon l'invention peut également comprendre un ou plusieurs tensioactifs, choisis de préférence parmi les émulsionnants eau-dans-huile (E/H) ou huile-dans-eau (H/E).

L'émulsionnant huile-dans-eau (H/E) peut notamment être choisi parmi les esters de sorbitane éventuellement polyéthoxylés, les esters d'acides gras et de glycérol, les esters ou polyesters d'acides gras et de sucrose, les esters d'acides gras et de polyéthylèneglycol, les polysiloxanes modifiés polyéthers, les éthers d'alcools gras et de polyéthylèneglycol, les alkylpolyglycosides et la lécithine hydrogénée, sans que cette liste ne soit limitative.

L'émulsionnant eau-dans-huile (E/H) peut être choisi parmi les esters gras non éthoxylés de polyols, et notamment parmi les esters gras non éthoxylés de glycérol, de polyglycérols, de sorbitol, de sorbitan, d'anydrodrohexitols comme en particulier l'isosorbide, de mannitol, de xylitol, d'érythritol, de maltitol, de saccharose, de glucose, de polydextrose, de sirops de glucose hydrogéné, de dextrines et d'amidons hydrolysés.

L'émulsionnant E/H peut être choisi parmi les esters gras non éthoxylés de polyols obtenus à partir d'acide gras ou par trans-estérification à partir d'huile ou de mélanges d'huiles. Les acides gras utilisés comprennent de 8 à 22 atomes de carbone, de préférence de 10 à 18 atomes de carbone, et en particulier de 12 à 18 atomes de carbone. Ces acides peuvent être linéaires ou ramifiés, saturés ou insaturés, posséder une ou des fonctions hydroxyles latérales. Les huiles peuvent être saturées ou insaturées, de liquide à solide à température ambiante, et posséder éventuellement des fonctions hydroxyle, de préférence d'indice d'iode compris entre 1 et 145, et en particulier de 5 à 105.

L'émulsionnant E/H peut être avantageusement choisi parmi les systèmes émulsionnants composés d'une cyclodextrine et d'un émulsionnant eau-dans-huile d'origine naturelle, tels que le système émulsionnant commercialisé par Roquette Frères sous le nom Beauté by Roquette^{®} DS146.

### Matière colorante

La composition cosmétique selon l'invention peut encore comprendre au moins une matière colorante choisie parmi les colorants hydrosolubles ou liposolubles, les charges ayant pour effet de colorer et/ou opacifier la composition et/ou de colorer les lèvres, telles que les pigments, les nacres, les laques (colorants hydrosolubles adsorbés sur un support minéral inerte) et leurs mélanges. Ces matières colorantes peuvent être éventuellement traitées en surface par un agent hydrophobe tel que les silanes, silicones, savons d'acides gras, C9-15 fluoroalcool phosphates, copolymères acrylate/dimethicone, copolymères mixtes C9-15 fluoroalcool phosphates / silicones, lécithines, cire de carnauba, polyéthylène, chitosan et acides aminés éventuellement acylés tels que la lauroyl lysine, le disodium stearoyl glutamate et l'aluminium acyl glutamate. Les pigments peuvent être minéraux ou organiques, naturels ou de synthèse. Des exemples de pigments sont notamment les oxydes de fer, de titane ou de zinc, ainsi que les pigments composites et les pigments goniochromatiques, perlescents, interférentiels, photochromes ou thermochromes, sans que cette liste ne soit limitative.

### Charges

La composition cosmétique selon l'invention peut en outre renfermer au moins une charge. Par ce terme, on entend toute particule de forme quelconque (notamment sphérique ou lamellaire), minérale ou organique, insoluble dans la composition. Des exemples de charges sont le talc, le mica, la silice, le kaolin, le nitrure de bore, l'amidon, l'amidon modifié par l'anhydride octénylsuccinique, les polyamides, les résines de silicone, les poudres d'élastomères de silicone et les poudres de polymères acryliques, en particulier de poly(méthacrylate de méthyle) ou les poudres de copolymère styrène acrylate (Sunsphere Powders de Dow).

### Applications cosmétiques

Selon un mode de réalisation, la composition cosmétique de soin est sous forme de masque. La présente invention concerne ainsi une composition cosmétique de soin de type masque comprenant dans un milieu physiologiquement acceptable, une composition filmogène comprenant au moins un amidon de légumineuse hydrolysé et alkylé, au moins un polyol, au moins un carraghénane.

Selon une notion bien connue de l'homme de l'art, on entend par composition de type masque une composition cosmétique formulée pour être appliquée sur les matières kératinique, préférentiellement la peau en une couche plus ou moins épaisse. Les compositions de type masque sont destinées à être laissées sur les matières kératiniques pendant une durée déterminée, par exemple pouvant être de quelques minutes à plusieurs dizaines de minute. Les compositions cosmétique de type masque pourront être laissées sur les matières kératiniques pendant une nuit entière pour un effet prolongé. A la fin de la durée prescrite, la composition de type masque peut être rincée à l'eau pour la décoller ou l'éliminer.

Il est du mérite de la Demanderesse d'avoir mis au point une composition cosmétique de type masque, grâce à la composition filmogène selon la présente invention, présentant, et d'excellentes propriétés mécaniques, notamment une bonne tenue mécanique, et une bonne aptitude à être éliminer postérieurement à l'application par lavage à l'eau de manière rapide et facilitée.

Selon un mode de réalisation, la présente invention concerne également une composition cosmétique de maquillage, de protection ou de soins des matières kératiniques comprenant une composition filmogène et au moins un ingrédient cosmétiquement actif, ladite composition filmogène comprenant au moins un amidon de légumineuse hydrolysé et alkylé, au moins un polyol, au moins un carraghénane.

On entend par ingrédient cosmétiquement actif ou substance cosmétiquement active tout ingrédient ou substance ayant une activité antivieillissement, une activité hydratante, un effet tenseur, une activité dépigmentant, une activité éclaircissante de la peau, un effet matifiant, une activité apaisante, une activité stimulant la microcirculation, une activité sébo-régulatrice, une activité de desquamation de l'épiderme, un ingrédient ou substance destiné à nettoyer ou purifier la peau, un effet tenseur.

Ainsi, outre la composition filmogène précédemment décrite, la composition cosmétique selon l'invention peut, par ailleurs, comprendre un ou plusieurs actifs hydrophiles ou lipophiles. En particulier, les agents actifs additionnels peuvent être choisis dans le groupe constitué des vitamines, des antioxydants, des agents anti-âges, des agents hydratants, des agents antipollution, les agents kératolytiques, des astringents, des anti-inflammatoires, des agents blanchissants et des agents favorisant la microcirculation, des agents sébo-régulateurs, des agents favorisant la desquamation de l'épiderme, des agents ayant un effet tenseur.

De préférence, outre la composition filmogène précédemment décrite, la composition cosmétique selon l'invention peut comprendre au moins un actif choisi parmi les agents anti-âge ou antivieillissement, les agents favorisant la desquamation de l'épiderme, les agents sébo-régulateurs, les agents ayant un effet tenseur et leurs mélanges, comme par exemple la gluconolactone ou la cyclodextrine hydroxypropylée.

Toute technique permettant d'incorporer un ingrédient cosmétiquement actif dans la composition filmogène pourra être utilisée par l'homme du métier.

Typiquement, dans le cas d'une composition cosmétique de type masque, l'ingrédient actif pourra être incorporé dans la composition filmogène afin d'obtenir l'effet cosmétique souhaité lors de l'application de la composition cosmétique de type masque sur les matières kératiniques, préférentiellement la peau et les cheveux.

La gluconolactone est un actif connu pour ses propriétés sébo-régulatrices, anti-âge ou antivieillissement et de desquamation de l'épiderme ou « peeling ».

Typiquement, la gluconolactone pourra être incorporée à la composition filmogène afin de conférer à la composition cosmétique ainsi obtenue des propriétés sébo-régulatrices, anti-âge ou antivieillissement et de desquamation de l'épiderme.

Typiquement, une cyclodextrine hydroxypropylée pourra être incorporée à la composition filmogène afin de conférer à la composition cosmétique obtenue un effet tenseur, et de propriétés anti-rides

La présente invention concerne également un procédé d'obtention d'un film cosmétique de type masque à partir d'une composition filmogène caractérisé en ce qu'il comprend les étapes de :
- fourniture d'une composition filmogène ;
- chauffage de ladite composition à une température comprise entre 40 et 90, préférentiellement 70 et 80°C ;
- séchage à une température comprise entre 20 et 50°C et entre 10 et 50% d'humidité relative.

La composition filmogène fournie est telle que précédemment décrite.
La présente invention concerne également un procédé de maquillage, de soin, ou de protection des matières kératiniques comprenant l'application sur lesdites matières kératiniques d'au moins une composition cosmétique selon la présente invention ;
La présente invention concerne également un procédé de maquillage, de protection ou de soin des matières kératiniques comprenant l'application sur lesdites matières kératiniques d'au moins une composition filmogène selon la présente invention.

L'invention a également pour objet l'utilisation d'une composition filmogène selon la présente invention pour la formation d'un film élastique.

L'utilisation non thérapeutique de la composition cosmétique conforme à l'invention comprend tous les soins du corps et de la peau, notamment les masques, y compris les produits assurant l'amélioration de l'aspect de la peau. L'utilisation non thérapeutique conforme à l'invention comprend également les produits de maquillage tels que les vernis, les mascaras, les rouges à lèvres, ou les produits capillaires tels que les shampoings, après-shampoings, soins, masques, huiles capillaires réparatrices et/ou protectrices. Avantageusement, la composition filmogène selon la présente invention pourra être incorporée dans tous les soins du corps et de la peau ainsi que dans tous les produits de maquillage précédemment cités.

L'utilisation non thérapeutique de la composition cosmétique conforme à l'invention comprend aussi toutes les protections de la peau par effet barrière ou effet seconde peau, contre les rayonnements du soleil, notamment contre les rayons ultra-violets.

L'invention est également décrite par l'intermédiaire des exemples ci-après qui se veulent purement illustratifs et ne limitent en rien la portée de la présente invention.

### Exemples

Trois compositions filmogènes selon l'invention sont préparées selon les compositions des tableaux 1 à 3.

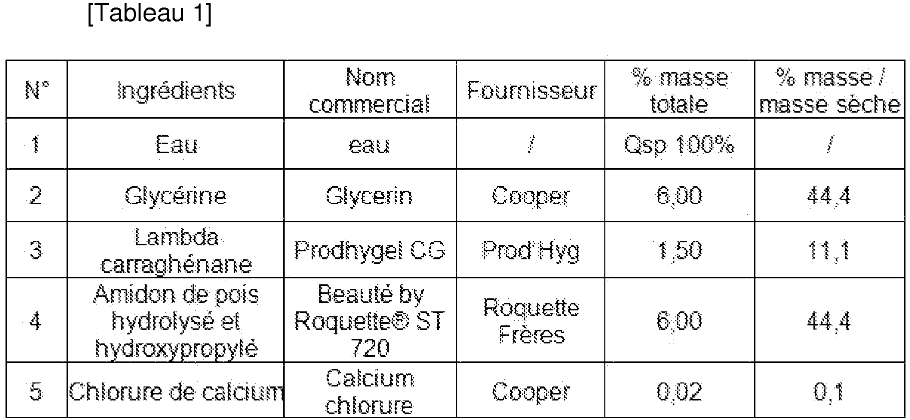

Le tableau 1 correspond à la composition filmogène A.

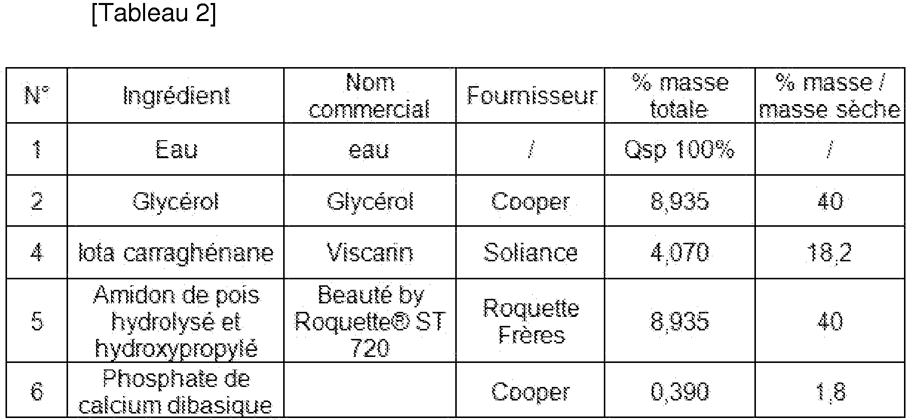

Le tableau 2 correspond à la composition filmogène B.

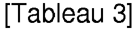

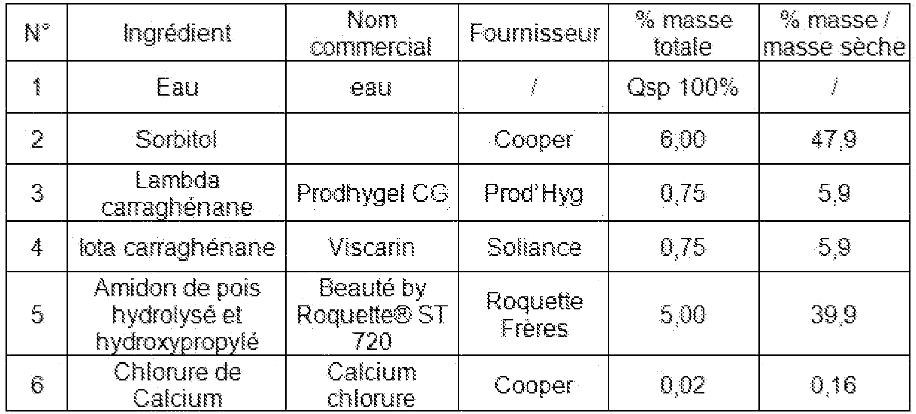

Le tableau 3 correspond à la composition filmogène C.

Le protocole de préparation des compositions A, B et C est le suivant :
On chauffe un bain-marie à une température de 75°C.

On ajoute un à un tous les ingrédients en attendant la dissolution complète d'un ingrédient avant d'ajouter le suivant, en suivant l'ordre numérique des tableaux 1 à 3.

On ajuste la température de la solution obtenue à l'étape précédente à une température de 72°C.

### Exemple 2

Les compositions filmogènes A, B et C sont utilisées pour préparer des films à appliquer sur la peau. Pour ce faire, pour chaque composition filmogène, on coule la solution homogène obtenue à l'exemple 1 sur un support plat en verre approprié, puis on attend quelques minutes que la gélification de la solution ait lieu. On fait ensuite sécher en étuve à 40°C sous 30% d'humidité relative pendant au moins 24 heures. A l'issue de ce protocole, on obtient un film de résistance mécanique suffisante pour être appliqué sur la peau, et de solubilité dans l'eau suffisante pour être dissout par lavage à l'eau.

### Exemple 3

Un masque de soin anti-âge, formé avec la composition filmogène selon l'invention, a été préparé selon la formule du tableau 4.

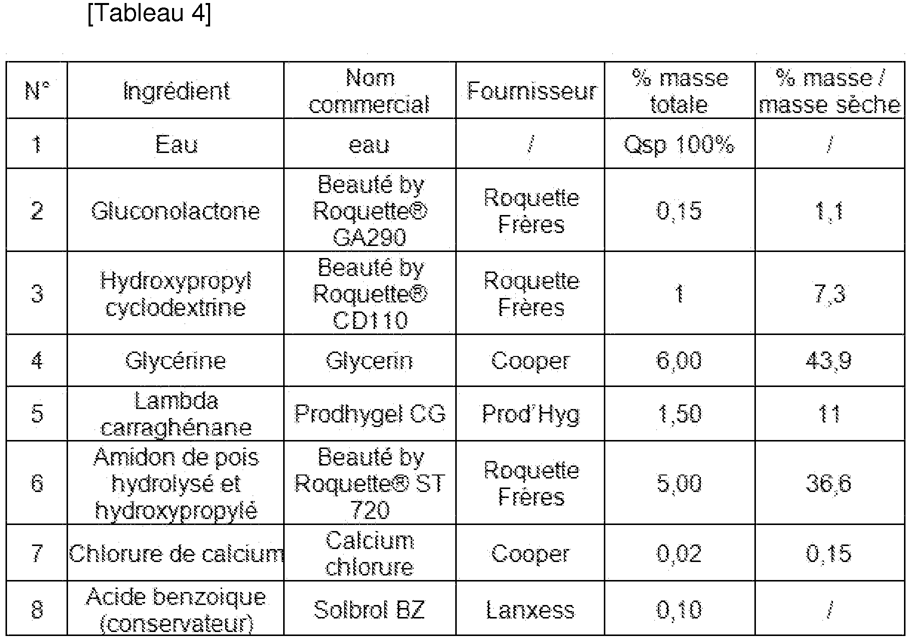

Pour préparer le masque, on procède premièrement à la dissolution de la gluconolactone dans la totalité du volume d'eau nécessaire à 45°C sous agitation. On ajoute ensuite la glycérine, le lambda carraghénane et l'amidon de pois hydrolysé et hydroxypropylé successivement, en attendant la dissolution complète entre chaque ajout à 45°C. On chauffe ensuite à 80°C, puis on ajoute le chlorure de calcium, et on agite jusqu'à dissolution complète.

La composition de masque préparée précédemment est ensuite coulée sur un support en verre plat de façon à obtenir une épaisseur d'environ 3 mm. On fait ensuite sécher en étuve à 40°C sous 30% d'humidité relative pendant au moins 24 heures.

On obtient alors un film de bonne résistance mécanique. On l'applique sur la peau pendant au moins une nuit. On ressent un effet de tension sur la peau. Après retrait du masque par lavage de la peau à l'eau, on constate que la peau est plus douce, et paraît retendue.

## Revendications

1. Composition filmogène comprenant de
- 1 % à 20 % d'au moins un amidon de légumineuse hydrolysé et alkylé,
- 1 % à 20% d'au moins un polyol, et
- 1 % à 10 % d'au moins un carraghénane,
les pourcentages étant exprimés en poids par rapport au poids total de la composition filmogène.

2. Composition selon la revendication 1, **caractérisée en ce que** ledit amidon de légumineuse est un amidon hydrolysé et hydroxypropylé.

3. Composition selon la revendication 2, **caractérisé en ce que** la teneur en groupements hydroxypropyle est comprise entre 0,1 et 20% en poids sec par rapport au poids sec d'amidon hydroxypropylé, préférentiellement entre 1 et 10%.

4. Composition selon l'une quelconque des revendications 1 à 3, **caractérisée en ce que** ledit au moins un amidon de légumineuse est un amidon de pois.

5. Composition selon l'une quelconque des revendications 1 à 4, **caractérisée en ce que** ledit au moins un polyol est choisi parmi le groupe constitué du maltitol, mannitol, xylitol, érythritol, sorbitol, glycérol, glucose, saccharose, polydextrose, les sirops de glucose hydrogénés, les dextrines, les maltodextrines, les sirops de glucose, et leurs mélanges.

6. Composition selon l'une quelconque des revendications 1 à 5, **caractérisée en ce que** ledit au moins un carraghénane est choisi parmi le iota carraghénane, le lambda carraghénane, le kappa carraghénane, et leurs mélanges.

7. Composition cosmétique de maquillage, de protection ou de soin des matières kératiniques comprenant dans un milieu physiologiquement acceptable, une composition filmogène comprenant au moins un amidon de légumineuse hydrolysé et alkylé, au moins un polyol, et au moins un carraghénane.

8. Composition cosmétique selon la revendication 7 **caractérisée en ce que** ladite composition filmogène comprend :
- 1 % à 20 % d'au moins un amidon de légumineuse hydrolysé et alkylé,
- 1 % à 20% d'au moins un polyol, et
- 1 % à 10 % d'au moins un carraghénane,
les pourcentages étant exprimés en poids par rapport au poids total de la composition filmogène.

9. Procédé de maquillage, de protection ou de soin des matières kératiniques comprenant l'application sur lesdites matières kératiniques d'au moins une composition cosmétique selon les revendications 7 et 8.

10. Utilisation non thérapeutique d'une composition filmogène selon les revendications 1 à 6 destinée à conférer aux matières kératiniques au moins une propriété choisie parmi l'unification du teint, l'uniformité du teint, l'homogénéité du teint, l'obtention d'un teint matifié, un effet barrière, le lissage de la peau, la protection des zones sensibilisées d'une fibre capillaire, le lissage des écailles des fibres capillaires, le gainage des fibres capillaires, le recourbement et l'allongement des cils, la résistance à des chocs mécaniques

## Patentansprüche

1. Filmbildende Zusammensetzung, umfassend
- 1 % bis 20 % mindestens einer hydrolysierten und alkylierten Hülsenfruchtstärke,
- 1 % bis 20 % mindestens eines Polyols und
- 1 % bis 10 % mindestens eines Carrageenans,
wobei die Prozentangaben in Gewichtsprozent ausgedrückt sind, bezogen auf das Gesamtgewicht der filmbildenden Zusammensetzung.

2. Zusammensetzung nach Anspruch 1, **dadurch gekennzeichnet, dass** die Hülsenfruchtstärke eine hydrolysierte und hydroxypropylierte Stärke ist.

3. Zusammensetzung nach Anspruch 2, **dadurch gekennzeichnet, dass** der Gehalt an Hydroxypropyl-Gruppen zwischen einschließlich 0,1 und 20 Trockengewichts-% beträgt, bezogen auf das Trockengewicht der hydroxypropylierten Stärke, vorzugsweise zwischen 1 und 10%.

4. Zusammensetzung nach einem der Ansprüche 1 bis 3, **dadurch gekennzeichnet, dass** die mindestens eine Hülsenfruchtstärke eine Erbsenstärke ist.

5. Zusammensetzung nach einem der Ansprüche 1 bis 4, **dadurch gekennzeichnet, dass** das mindestens eine Polyol aus der Gruppe ausgewählt ist, die aus Maltitol, Mannitol, Xylitol, Erythritol, Sorbitol, Glycerin, Glukose, Saccharose, Polydextrose, hydrierten Glukosesirupen, Dextrinen, Maltodextrinen, Glukosesirupen und deren Mischungen besteht.

6. Zusammensetzung nach einem der Ansprüche 1 bis 5, **dadurch gekennzeichnet, dass** das mindestens eine Carrageenan aus lota-Carrageenan, Lambda-Carrageenan, Kappa-Carrageenan und deren Mischungen ausgewählt ist.

7. Kosmetische Zusammensetzung zum Schminken, zum Schutz oder zur Pflege von Keratinmaterialien, die in einem physiologisch akzeptablen Medium eine filmbildende Zusammensetzung umfasst, die mindestens eine hydrolysierte und alkylierte Hülsenfruchtstärke, mindestens ein Polyol und mindestens ein Carrageenan umfasst.

8. Kosmetische Zusammensetzung nach Anspruch 7, **dadurch gekennzeichnet, dass** die filmbildende Zusammensetzung umfasst:
- 1 % bis 20 % mindestens einer hydrolysierten und alkylierten Hülsenfruchtstärke,
- 1 % bis 20 % mindestens eines Polyols und
- 1 % bis 10 % mindestens eines Carrageenans,
wobei die Prozentangaben in Gewichtsprozent ausgedrückt sind, bezogen auf das Gesamtgewicht der filmbildenden Zusammensetzung.

9. Verfahren zum Schminken, zum Schützen oder zum Pflegen von Keratinmaterialien, welches das Auftragen mindestens einer kosmetischen Zusammensetzung nach den Ansprüchen 7 und 8 auf die Keratinmaterialien umfasst.

10. Nicht therapeutische Verwendung einer filmbildenden Zusammensetzung nach den Ansprüchen 1 bis 6, die dazu bestimmt ist, den Keratinmaterialien mindestens eine Eigenschaft zu verleihen, die ausgewählt ist aus einem ebenmäßigen Teint, einem gleichmäßigen Teint, einem homogenen Teint, einem mattierten Teint, einer Barrierewirkung, der Glättung der Haut, dem Schutz der sensibilisierten Bereiche einer Haarfaser, der Glättung der Schuppen der Haarfasern, die Ummantelung der Haarfasern, dem Schwung und der Verlängerung der Wimpern, der Widerstandsfähigkeit gegenüber mechanischen Belastungen.

## Claims

1. A film-forming composition comprising
- from 1% to 20% of at least one hydrolysed and alkylated legume starch,
- from 1% to 20% of at least one polyol, and
- from 1% to 10% of at least one carrageenan,
the percentages being expressed by weight relative to the total weight of the film-forming composition.

2. The composition according to claim 1, **characterised in that** said legume starch is a hydrolysed and hydroxypropylated starch.

3. The composition according to claim 2, **characterised in that** the content of hydroxypropyl groups is between 0.1 and 20%, preferably between 1 and 10%, by dry weight relative to the dry weight of hydroxypropylated starch.

4. The composition according to any of claims 1 to 3, **characterised in that** said at least one legume starch is a pea starch.

5. The composition according to any of claims 1 to 4, **characterised in that** said at least one polyol is selected from the group consisting of maltitol, mannitol, xylitol, erythritol, sorbitol, glycerol, glucose, sucrose, polydextrose, hydrogenated glucose syrups, dextrins, maltodextrins, glucose syrups, and mixtures thereof.

6. The composition according to any of claims 1 to 5, **characterised in that** said at least one carrageenan is selected from iota carrageenan, lambda carrageenan, kappa carrageenan and mixtures thereof.

7. A cosmetic composition for making up, protecting or caring for keratin materials, comprising, in a physiologically acceptable medium, a film-forming composition comprising at least one hydrolysed and alkylated legume starch, at least one polyol and at least one carrageenan.

8. The cosmetic composition according to claim 7, **characterised in that** said film-forming composition comprises:
- from 1% to 20% of at least one hydrolysed and alkylated legume starch,
- from 1% to 20% of at least one polyol, and
- from 1% to 10% of at least one carrageenan,
the percentages being expressed by weight relative to the total weight of the film-forming composition.

9. A method for making up, protecting or caring for keratin materials, comprising applying at least one cosmetic composition according to claims 7 and 8 to said keratin materials.

10. A non-therapeutic use of a film-forming composition according to claims 1 to 6 for providing the keratin materials with at least one property selected from unifying the complexion, uniformity of the complexion, homogeneity of the complexion, obtaining a mattified complexion, a barrier effect, smoothing the skin, protecting sensitised zones of a hair fibre, smoothing scales of hair fibres, sheathing the hair fibres, curling and lengthening the eyelashes, mechanical shock resistance.
